Europäisches Patentamt

European Patent Office

Office européen des brevets

(11) Veröffentlichungsnummer: **0 576 066 A2**

# EUROPÄISCHE PATENTANMELDUNG

(21) Anmeldenummer: **93201688.4**

(22) Anmeldetag: **11.06.93**

(51) Int. Cl.5: **A61B 6/00**, A61B 6/04,
H04N 5/32

(30) Priorität: **20.06.92 DE 4220282**

(43) Veröffentlichungstag der Anmeldung:
**29.12.93 Patentblatt 93/52**

(84) Benannte Vertragsstaaten:
**DE FR GB IT NL**

(71) Anmelder: **Philips Patentverwaltung GmbH**
**Wendenstrasse 35c**
**D-20097 Hamburg(DE)**

(84) **DE**

(71) Anmelder: **PHILIPS ELECTRONICS N.V.**

Groenewoudseweg 1
NL-5621 BA Eindhoven(NL)

(84) **FR GB IT NL**

(72) Erfinder: **Haaks, Winfried, Dr.**
**c/o Philips Patentverwaltung GmbH,**
**Wendenstr. 35**
**D-20097 Hamburg(DE)**

(74) Vertreter: **Hartmann, Heinrich, Dipl.-Ing.**
**Philips Patentverwaltung GmbH,**
**Wendenstrasse 35c**
**D-20097 Hamburg (DE)**

(54) **Verfahren für die periphere Angiographie und Anordnung zur Durchführung des Verfahrens.**

(57) Die Erfindung betrifft ein Verfahren für die periphere Angiographie, bei dem der Untersuchungsbereich und ein Röntgenaufnahmesystem relativ zueinander verschoben werden, wobei in unterschiedlichen Positionen Röntgenaufnahmen benachbarter Teile des Untersuchungsbereichs erstellt werden, wobei aus den Röntgenaufnahmen ein Kontrastmittelbild und ein Maskenbild abgeleitet und das Kontrastmittelbild und das Maskenbild voneinander subtrahiert werden, sowie eine Anordnung zur Durchführung des Verfahrens. Eine verbesserte geometrische Übereinstimmung zwischen dem Kontrastmittelbild und dem Maskenbild ergibt sich erfindungsgemäß dadurch, daß nach Kontrastmittelinjektion die Verschiebung so gesteuert wird, daß der Kontrastmittelbolus jeweils in den Röntgenaufnahmen abgebildet wird, und daß aus den das Kontrastmittel bereits darstellenden Zeilen der Röntgenaufnahmen das Kontrastmittelbild und aus den das Kontrastmittel noch nicht darstellenden Teilen der Röntgenaufnahmen das Maskenbild abgeleitet wird.

Fig.1

EP 0 576 066 A2

Die Erfindung betrifft ein Verfahren für die periphere Angiographie, bei dem der Untersuchungsbereich und ein Röntgenaufnahmesystem relativ zueinander verschoben werden, wobei in unterschiedlichen Positionen Röntgenaufnahmen benachbarter Teile des Untersuchungsbereichs erstellt werden, wobei aus den Röntgenaufnahmen ein Kontrastmittelbild und ein Maskenbild abgeleitet werden und wobei das Kontrastmittelbild und das Maskenbild voneinander subtrahiert werden, sowie eine Anordnung zur Durchführung des Verfahrens.

Ein solches Verfahren und eine solche Anordnung sind aus der DE-PS 39 19 473 bekannt. Das Kontrastmittelbild und das Maskenbild werden in aufeinanderfolgenden Arbeitsgängen erhalten. Zur Gewinnung des Maskenbildes werden benachbarte Abschnitte, beispielsweise eines Beines, aufgenommen, ohne daß ein Kontrastmittel injiziert worden ist. In einem zweiten Arbeitsgang werden zur Gewinnung eines Kontrastmittelbildes nach Injektion eines Kontrastmittels von den gleichen Abschnitten Röntgenaufnahmen angefertigt.

Im Idealfall unterscheiden sich das Kontrastmittelbild und das Maskenbild nur dadurch, daß im Kontrastmittelbild das Gefäßsystem durch das injizierte Kontrastmittel hervorgehoben ist. Durch Subtraktion der beiden Bilder voneinander wird ein Bild des Gefäßsystems erhalten, bei dem die überlagerten Strukturen, beispielsweise Knochen, eliminiert sind.

Der Vorteil eines solchen Subtraktionsangiographieverfahrens gegenüber einer konventionellen Angiographie besteht darin, daß man mit einer geringen Kontrastmittelkonzentration kontrastreiche (Subtraktions-) Bilder erzeugen kann. Ein Nachteil des bekannten Verfahrens besteht darin, daß es zu einer ungenügenden geometrischen Übereinstimmung zwischen dem Maskenbild und dem Kontrastmittelbild kommen kann, wenn der Patient sich in dem Zeitraum zwischen der Erstellung der Röntgenaufnahmen für das Maskenbild und der Röntgenaufnahmen für das Kontrastmittelbild bewegt. Eine solche nicht reproduzierbare Bewegung kann sich schon daraus ergeben, daß der Patient bzw. die ihn tragende Lagerungsplatte des Patientenlagerungstisches in die verschiedenen Aufnahmepositionen verschoben wird.

Aufgabe der Erfindung ist es, ein Verfahren anzugeben, bei dem besser gewährleistet ist, daß das Kontrastmittelbild und das Maskenbild übereinstimmen. Diese Aufgabe wird erfindungsgemäß dadurch gelöst, daß nach Kontrastmittelinjektion die Verschiebung so gesteuert wird, daß der Kontrastmittelbolus jeweils in den Röntgenaufnahmen abgebildet wird, und daß aus den das Kontrastmittel bereits darstellenden Zeilen der Röntgenaufnahmen das Kontrastmittelbild und aus den das Kontrastmittel noch nicht darstellenden Teilen der Röntgenaufnahmen das Maskenbild abgeleitet wird.

Wenn der Kontrastmittelbolus, d.h. der in Ausbreitungsrichtung vorderste Teil des Kontrastmittels, in jeder Röntgenaufnahme abgebildet wird, dann liefert das Röntgenaufnahmesystem stets Zeilen, in denen das Kontrastmittel abgebildet ist und Zeilen, in denen es - noch - nicht abgebildet ist.

Bei der Erfindung werden die von dem Röntgenaufnahmesystem gelieferten Bildzeilen, in denen das Kontrastmittel schon erkennbar ist, zur Erzeugung des Kontrastmittelbildes herangezogen, und die Bildzeilen, die noch kein Kontrastmittel zeigen, zur Erzeugung des Maskenbildes. Kontrastmittelbild und Maskenbild werden also aus Bildzeilen zusammengesetzt, die aus derselben Röntgenaufnahme oder aus aufeinanderfolgenden Röntgenaufnahmen stammen. Wegen dieses geringen bzw. nicht vorhandenen zeitlichen Unterschiedes bei diesen Aufnahmen ist die Gefahr, daß infolge von Bewegungen des Patienten Kontrastmittelbild und Maskenbild geometrisch nicht mehr übereinstimmen, deutlich verringert. Zudem ist das Verfahren schneller, weil Kontrastmittelbild und Maskenbild aus einer einzigen Serie von Röntgenaufnahmen abgeleitet werden können.

Eine Anordnung zur Durchführung des erfindungsgemäßen Verfahrens, die versehen ist mit einem Patientenlagerungstisch und einem Röntgenaufnahme-System zur Erzeugung von Röntgenaufnahmen sowie einem Antrieb zum Verschieben des Patientenlagerungstisches relativ zum Röntgenaufnahmesystem ist gekennzeichnet durch

- Steuermittel zum Steuern des Aufnahmezeitpunktes und/oder des Antriebs derart, daß der Kontrastmittelbolus in den Röntgenaufnahmen abgebildet wird
- einen ersten Speicher zur Aufnahme der Zeilen ohne Kontrastmittel und einen zweiten Speicher zur Aufnahme von Zeilen mit Kontrastmittel und durch
- eine Einheit, die aus den Bildzeilen im ersten bzw. im zweiten Bildspeicher das Maskenbild bzw. das Kontrastmittelbild erstellt.

Eine Weiterbildung der Erfindung sieht vor, daß eine Wiedergabeeinheit zur Beobachtung des Kontrastmittelflusses vorgesehen ist und daß Mittel zum Steuern des Antriebs und/oder des Aufnahmezeitpunkts durch den Benutzer vorgesehen sind. Diese Ausbildung erlaubt es dem Benutzer, den Aufnahmezeitpunkt (bei vorgegebener Geschwindigkeit der Relativverschiebung zwischen Röntgenaufnahmesystem und Untersuchungsbereich), die Geschwindigkeit (bei vorgegebenem Zeitpunkt) oder beides zusammen unter Beobachtung des Kontrastmittelflusses so zu steuern, daß der Kontrastmittelbolus, d.h. der in Ausbreitungsrichtung vorderste Teil des Kontrastmittels in der Röntgenaufnahme vorzugsweise in ihrem - in Ausbreitungsrichtung gesehen - mittleren Teil abgebildet wird.

Wenn dabei die Verschiebungsrichtung zumindest näherungsweise senkrecht zur Richtung der Zeilen verläuft, dann ist der Bereich des Patienten, der bei einer (ersten) Röntgenaufnahme abgebildet und bei der nachfolgenden (zweiten) Röntgenaufnahme nicht mehr abgebildet wird, mit Kontrastmittel gefüllt. Die diesem Bereich zugeordneten Zeilen der ersten Röntgenaufnahme werden in dem Speicher für das Kontrastmittelbild gespeichert. Entsprechend wird die gleiche Zeilenzahl von der zweiten Röntgenaufnahme, die dem von der ersten Röntgenaufnahme noch nicht erfaßten Bereich zugeordnet sind, in dem Speicher für das Maskenbild gespeichert, denn diesen Bereich hat das Kontrastmittel bei der zweiten Röntgenaufnahme noch nicht erreicht. Bildinterpretationsmittel sind dabei nicht erforderlich, was die Anordnung verbilligt und vereinfacht.

Nach einer anderen Ausgestaltung der Erfindung ist vorgesehen, daß Mittel zum Bestimmen der Kontrastmittelgeschwindigkeit aus aufeinanderfolgenden Röntgenaufnahmen und zum Bestimmen des Aufnahmezeitpunktes und/oder der die Geschwindigkeit des Antriebs für die nachfolgende Röntgenaufnahme vorgesehen sind. Die Kontrastmittelgeschwindigkeit läßt sich aus den beiden letzten Röntgenaufnahmen bestimmen, wenn die Verschiebung des Kontrastmittelbolus durch den zeitlichen Abstand der beiden letzten Aufnahmen dividiert wird. Die Geschwindigkeit des Kontrastmittelbolus ergibt sich aus der Verschiebung des Kontrastmittelbolus innerhalb dieser Aufnahmen und der Relativverschiebung zwischen dem Untersuchungsbereich und dem Röntgenaufnahmesystem - jeweils zwischen der vorletzten und der letzten Aufnahme. Diese Parameter lassen sich durch Bildinterpretationsmittel zur Detektion der Kontrastmittelgrenze ermitteln, so daß der Aufnahmezeitpunkt bzw. die Tischplattengeschwindigkeit automatisch der Kontrastmittelgeschwindigkeit angepaßt werden kann. Wenn die Röntgenaufnahmen mit einer konstanten Aufnahmefrequenz erzeugt werden, muß lediglich die Geschwindigkeit der Relativverschiebung zwischen Untersuchungsbereich und Röntgenaufnahmesystem geregelt werden bzw. die Größe der Verschiebung bis zur nächsten Röntgenaufnahme.

Um die Relativverschiebung zwischen Untersuchungsbereich und Röntgenaufnahmesystem bei aufeinanderfolgenden Röntgenaufnahmen genau bestimmen zu können, ist in Ausbildung der Erfindung vorgesehen, daß im Strahlengang des Röntgenaufnahmesystems Marken vorgesehen sind, die in einer Röntgenaufnahme abbildbar sind. Die Marken können beispielsweise in der Tischplatte vorgesehen sein, auf der der Patient gelagert ist. Ihre Lage in den Röntgenaufnahmen läßt sich bei geeigneter Formgebung leicht mit Hilfe eines automatischen Auswerteverfahrens bestimmen und zur geometrisch korrekten Ableitung des Maskenbildes bzw. des Kontrastmittelbildes aus Teilen von jeder der Röntgenaufnahmen nutzen.

Die Erfindung wird nachstehend anhand der Zeichnungen erläutert. Es zeigen

Fig. 1 eine Röntgenuntersuchungseinrichtung für die periphere Angiographie,

Fig. 2 einen Teil dieser Einrichtung und

Fig. 3a und 3b zwei aufeinanderfolgende Röntgenaufnahmen des Gefäßsystems.

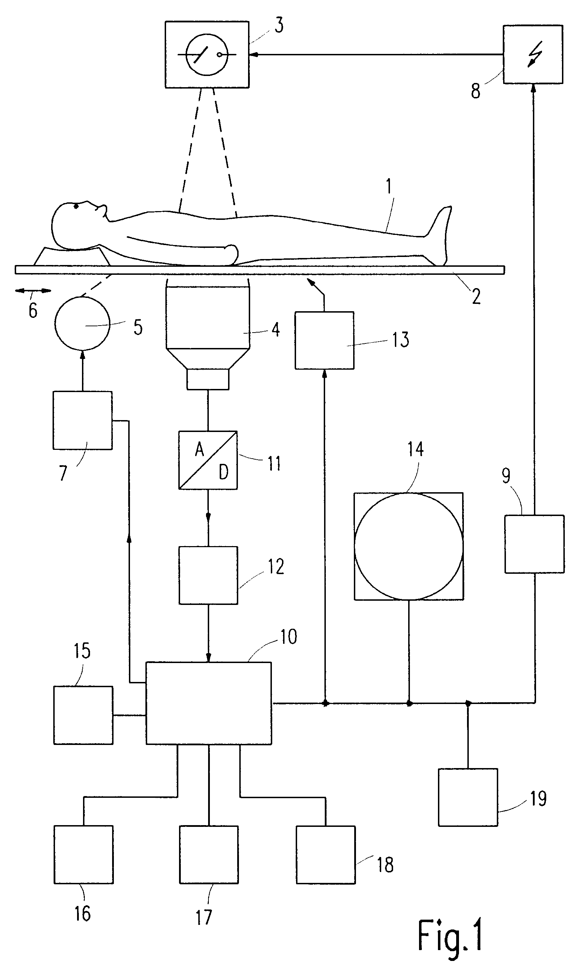

In Fig. 1 ist mit 1 ein zu untersuchender Patient bezeichnet, der auf einem Patientenlagerungstisch gelagert ist, von dem lediglich die Tischplatte 2 dargestellt ist. Der Untersuchungsbereich wird durch die Stellung der Tischplatte 2 in bezug auf ein Röntgenaufnahmesystem bestimmt, das aus einem Röntgenstrahler 3 und einem Röntgenbildwandler 4, beispielsweise einer Bildverstärker-Fernsehkette, besteht. Der Untersuchungsbereich ist gegenüber dem Röntgenaufnahmesystem 3, 4 verschiebbar. Zu diesem Zweck ist ein Motor 5 vorgesehen, der die Tischplatte 2 in ihrer Längsrichtung, d.h. in Richtung des Doppelpfeiles 6 verschieben kann. Der Motor 5 wird von einem Motorcontroller 7 gesteuert.

Der Röntgenstrahler 3 ist an einen Röntgengenerator 8 angeschlossen, der eine kontinuierliche Durchleuchtung gestattet, sowie Röntgenaufnahmen mit erhöhter Intensität. Der Zeitpunkt und die Parameter dieser Röntgenaufnahmen werden durch eine Belichtungseinheit 9 bestimmt, die ihrerseits von einer Bildverarbeitungs- und Steuereinheit 10 gesteuert wird.

Der Röntgenbildwandler 4 liefert elektrische Signale, die die Röntgenaufnahme bzw. das Röntgendurchleuchtungsbild zeilenweise wiedergeben. Die Zeilenrichtung verläuft dabei vorzugsweise senkrecht zur Tischlängsrichtung und somit senkrecht zur Zeichenebene. Das von dem Röntgenbildwandler 4 erzeugte analoge Signal wird von einem Analog-Digital-Wandler 11 in eine Folge digitaler Datenworte umgesetzt. Der so erzeugte Datenstrom wird über einen Zwischenspeicher 12, der die Zwischenspeicherung mehrerer Bildzeilen gestattet, der Bildverarbeitungs- und Steuereinheit 10 zugeführt.

Die Einheit 10 steuert u.a. den Motorcontroller 7 sowie einen Kontrastmittelinjektor 13, mit dessen Hilfe dem Patienten 1 ein Kontrastmittel injiziert werden kann. Ein an die Einheit 10 angeschlossener Monitor 14 gestattet es, die Ausbreitung des Kontrastmittels zu verfolgen. Mit einer an die Einheit 10 angeschlossenen Bedienungseinheit 15 kann der Benutzer verschiedene Untersuchungsparameter auch manuell vorgeben, beispielsweise die Geschwindigkeit der Tischplattenverschiebung oder die Auslösezeitpunkte für die Röntgenaufnahme.

Die Bildverarbeitungs- und Steuereinheit greift auf einen Bildspeicher 16 für ein Kontrastmittelbild und einen Bildspeicher 17 für ein Maskenbild zu. Subtraktionsbilder können in einem digitalen Archivspeicher 18 gespeichert oder an einer Filmausgabeeinheit 19 als Filmbild ausgegeben werden.

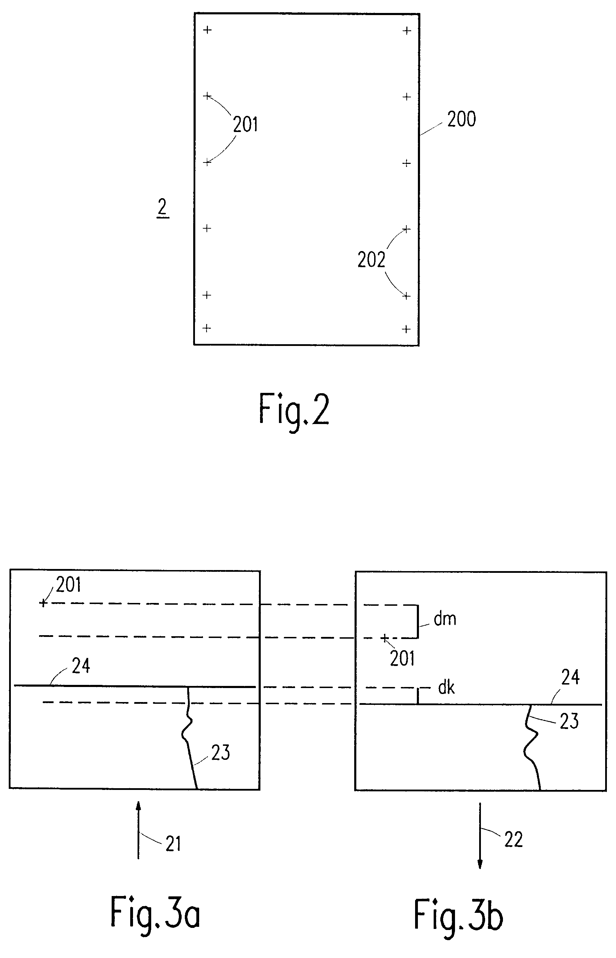

Fig. 2 entspricht einer Röntgenaufnahme der Tischplatte 2. Neben dem Rand 200 der Tischplatte erkennt man zwei Gruppen von die Röntgenstrahlung stark absorbierenden Marken 201 und 202, die auf zur Tischlängsrichtung parallelen Geraden angeordnet sind, wobei zweckmäßigerweise je eine Marke 201 und 202 eine zur Tischlängsrichtung senkrechte Zeile definieren. Die Marken können aus einem geeigneten Metall bestehen und sind so geformt, daß sie mit Hilfe einfacher Bildverarbeitungsalgorithmen schnell und zuverlässig in einer Röntgenaufnahme detektiert werden können. Sie sind so angeordnet, daß in allen Tischplattenpositionen mindestens eine Marke in der Röntgenaufnahme abgebildet wird.

Nachfolgend wird der Verlauf einer angiographischen Untersuchung beschrieben, wobei davon ausgegangen wird, daß das Eingangsformat des Röntgenbildwandlers 4 nicht ausreicht, um den gesamten zu untersuchenden Bereich (Bein) zu erfassen. Die Untersuchung beginnt mit der Injektion eines Kontrastmittels durch den Kontrastmittelinjektor 13. Sobald das Kontrastmittel sich auszubreiten beginnt, startet der Motorcontroller 7 die Verschiebung der Tischplatte in Richtung auf das Kopfende hin, so daß ein Bein des Patienten abschnittsweise bis zu den Füßen aufgenommen wird. Die Tischplattengeschwindigkeit wird der Ausbreitungsgeschwindigkeit des Kontrastmittels angepaßt, so daß im Idealfall der Kontrastmittelbolus seine Lage in den einzelnen Röntgenaufnahmen nicht ändert und vorzugsweise in der Mitte der Röntgenaufnahme abgebildet wird.

Die Röntgenaufnahmen erfolgen vorzugsweise mit einer konstanten Bildfrequenz. Diese muß so gewählt sein, daß auch bei der größten Ausbreitungsgeschwindigkeit des Kontrastmittels die Verschiebung zwischen zwei aufeinanderfolgenden Röntgenaufnahmen kleiner ist als die Hälfte der Höhe einer Röntgenaufnahme (die Höhe ist die Abmessung einer Röntgenaufnahme in der Verschiebungsrichtung). Bei einer niedrigeren Bildfrequenz können Lücken in dem Maskenbild oder in dem Kontrastmittelbild auftreten, die aus den Röntgenaufnahmen abgeleitet werden.

Figuren 3a und 3b zeigen zwei Aufnahmen, wobei die Aufnahme gemäß Fig. 3b der Aufnahme gemäß Fig. 3a unmittelbar folgt. In beiden Röntgenaufnahmen sind alle anatomischen Einzelheiten der besseren Übersicht halber weggelassen - bis auf ein mit Kontrastmittel gefülltes Gefäß 23. Die Ausbreitungsrichtung des Kontrastmittels ist durch den Pfeil 21 bezeichnet (Fig. 3a), während der entgegengerichtete Pfeil 22 (Fig. 3b) die Verschiebungsrichtung der Tischplatte angibt. Die Zeile, auf der sich der Kontrastmittelbolus befindet, ist in beiden Röntgenaufnahmen durch die durchzogene Linie 24 dargestellt.

Wenn angenommen wird, daß bei der Abtastung der einzelnen Röntgenaufnahmen die Zeilen von unten nach oben aufeinanderfolgen, dann liegen die ersten Zeilen in dem Teil der Röntgenaufnahme, in dem sich auch das mit Kontrastmittel gefüllte Gefäß 23 befindet. Diese Zeilen werden in den Speicher 16 für das Kontrastmittelbild eingelesen. Während des Einlesens der Zeilen prüft die Bildverarbeitungseinheit 10, ob sich in der betreffenden Zeile noch Kontrastmittel befindet, bzw. ein mit Kontrastmittel gefülltes Gefäß. Ist dies der Fall, dann wird die Zeile in den Speicher 16 eingelesen. Wenn die Kontrastmittelgrenze (Zeile 24) bzw. mehrere aufeinanderfolgende Zeilen ohne ein kontrastmittelgefülltes Gefäß detektiert sind, werden diese und alle weiteren Zeilen der Röntgenaufnahme in den Bildspeicher 17 für das Maskenbild geladen. Die geometrische Zuordnung wird dabei durch Detektion der Marke 201 in der Röntgenaufnahme erleichtert.

Die Detektion von Zeilen mit und ohne Kontrastmittel kann unter Anwendung bekannter Mustererkennungsverfahren erfolgen, die großflächigen Strukturen (Knochen) im Bild unterdrücken und Strukturen mit kleinen Abmessungen (z.B. Gefäße) anheben. Dabei kann zur Erhöhung der Sicherheit gegebenenfalls noch überprüft werden, ob die Positionen, die für ein kontrastmittelgefülltes Gefäß ermittelt worden sind, in beiden Zeilen annähernd gleich sind.

Entsprechend laufen die Vorgänge bei der Erfassung des nächsten Bildes (Fig. 3b) ab, wobei durch die Tischplattenbewegung am oberen (fußseitigen) Bildrand einige Zeilen hinzukommen, während am unteren (kopfseitigen) Bildrand die gleiche Anzahl von Zeilen entfällt.

Infolge der Tischplattenverschiebung zwischen den beiden Röntgenaufnahmen befindet sich die Abbildung der Marke 201 nicht mehr in derselben Zeile wie bei Fig. 3a, sondern in einer um dm Zeilen nach unten verschobenen Zeile. Die Zeile z in der Röntgenaufnahme gemäß Fig. 3a und die Zeile z-dm in der Röntgenaufnahme Fig. 3b (wobei die Zeilen von unten nach oben gezählt werden) betreffen also denselben anatomischen Bereich. Wenn diese beiden Zeilen in den beiden Einzelbildern nicht auf verschiedenen Seiten der Kontrastmittelgrenze 24 liegen, tauchen sie in einem der Bildspeicher doppelt auf. Deshalb wird entweder eine dieser Zeilen entfernt, oder - noch besser - Bildpunkt für Bildpunkt zu der anderen Zeile addiert, um eine Mittelwertbildung zu ermöglichen.

Die darauffolgenden Bilder werden entsprechend verarbeitet, so daß sich im Bildspeicher 17 allmählich ein Maskenbild des Beines (ohne Kontrastmittel) und im Bildspeicher 16 allmählich ein Kontrastmittelbild aufbaut.

Wie bereits erwähnt, ist es wichtig, daß die Zeile 24 (d.h. die Kontrastmittelgrenze) stets innerhalb des Bildes, vorzugsweise an einer festen Stelle, beispielsweise in der Bildmitte bleibt. Das kann dadurch erreicht werden, daß mit Hilfe des Monitors 14 der Benutzer unter Sichtkontrolle die Geschwindigkeit der Plattenverschiebung entsprechend variiert. Jedoch ist es auch möglich, die Geschwindigkeit automatisch anzupassen, indem die Kontrastmittelgeschwindigkeit zwischen einer Aufnahme und der ihr vorangehenden Aufnahme als Sollwert für die Verschiebegeschwindigkeit der Tischplatte bis zur nachfolgenden Röntgenaufnahme vorgegeben wird. Da die zeitlichen Abstände zwischen den Röntgenaufnahmen sich nicht verändern, ist es lediglich erforderlich, die absolute Verschiebung des Kontrastmittels aus den beiden aufeinanderfolgenden Aufnahmen zu bestimmen.

Wenn k1 und k2 in den Röntgenaufnahmen nach Fig. 3a und 3b die Zeilen bezeichnen, auf denen die Kontrastmittelgrenze 24 liegt, dann gilt für die absolute Verschiebung d (ausgedrückt in Zeilenzahlen)

$$d = dm - dk,$$

wobei dk = k1 - k2 ist. Die Bildverarbeitungs- und Steuereinheit 10 ermittelt aus der so berechneten absoluten Verschiebung d und dem Zeitraum zwischen zwei Röntgenaufnahmen die Verschiebegeschwindigkeit und gibt diese dem Motorcontroller 7 als Sollwert vor. In der Regel wird auch bei einer derartigen automatischen Geschwindigkeitsregelung die Kontrastmittelgrenze ihre Lage im Bild ändern, jedoch wird diese Lageänderung relativ klein sein, weil sich zwischen aufeinanderfolgenden Röntgenaufnahmen die Kontrastmittelgeschwindigkeit nur relativ wenig ändert.

Nachdem auf diese Weise in den Bildspeichern 16 und 17 ein Kontrastmittelbild bzw. ein Maskenbild erzeugt worden ist, läßt sich anschließend für einen beliebigen Abschnitt des Untersuchungsbereichs ein Subtraktionsbild berechnen, indem für den betreffenden Abschnitt Bildpunkt für Bildpunkt die Differenz zwischen dem Kontrastmittelbild und dem Maskenbild gebildet wird. Dieses kann dann auf dem Monitor 14 oder auf der Bildschirmausgabeeinheit 19 ausgegeben oder in den Archivspeicher 18 übernommen werden.

Wie sich aus dem Vorstehenden ergibt, sind die Marken 201 für die korrekte anatomische Zuordnung der einzelnen Zeilen wichtig. Anstelle der Marken könnte zu diesem Zweck aber auch ein Sensor verwendet werden, der die Tischplattenverschiebung mißt. Jedoch kann man auch ohne eine solche Positionsbestimmung auskommen, wenn man mit Hilfe des aus der DE-OS 41 02 729 bekannten Bildverarbeitungsverfahrens die aneinander anschließenden Bereiche aus überlappenden Röntgenaufnahmen identifiziert und zusammengefügt werden, und zwar getrennt für das Maskenbild und für das Kontrastmittelbild.

Das erfindungsgemäße Subtraktionsbild stellt ein statisches Bild der mit Kontrastmittel gefüllten Gefäße dar. Man kann aber auch in der üblichen Weise eine Darstellung der dynamischen Vorgänge erhalten, wenn man eine Folge der von dem AD-Wandler gelieferten, sich überlappenden Röntgenaufnahmen speichert. Es kann sich dabei um die gleichen Röntgenaufnahmen handeln, aus denen das Kontrastmittelbild, bzw. das Maskenbild abgeleitet wird, erforderlichenfalls aber auch um zusätzliche automatisch oder vom Benutzer in bestimmten Positionen erzeugte Röntgenaufnahmen. Der gestrichelt angedeutete Speicher 20 dient der Aufnahme dieser Bildfolge. Wenn die Bildverarbeitungs- und Steuereinheit 10 entsprechend ausgebildet ist, kann dieser Speicher - wie gestrichelt angedeutet, an diese Einheit angeschlossen sein; ansonsten wäre auch ein Anschluß an eine der Einheiten 11 oder 12 möglich.

**Patentansprüche**

1.  Verfahren für die periphere Angiographie, bei dem der Untersuchungsbereich (1,2) und ein Röntgenaufnahmesystem (3,4) zur Erzeugung von aus Zeilen zusammengesetzten Röntgenaufnahmen relativ zueinander verschoben werden, wobei in unterschiedlichen Positionen Röntgenaufnahmen benachbarter Teile des Untersuchungsbereichs erstellt werden, wobei aus den Röntgenaufnahmen ein Kontrastmittelbild und ein Maskenbild abgeleitet und das Kontrastmittelbild und das Maskenbild voneinander subtrahiert werden, dadurch gekennzeichnet, daß nach Kontrastmittelinjektion die Verschiebung so gesteuert wird, daß der Kontrastmittelbolus jeweils in den Röntgenaufnahmen abgebildet wird, und daß aus den das Kontrastmittel bereits darstellenden Zeilen der Röntgenaufnahmen das Kontrastmittelbild und aus den das Kontrastmittel noch nicht darstellenden Teilen der Röntgenaufnahmen das Maskenbild abgeleitet wird.

**2.** Anordnung zur Durchführung des Verfahrens nach Anspruch 1, mit einem Patientenlagerungstisch (2), einem Röntgenaufnahmesystem (3,4) zur Erzeugung von Röntgenaufnahmen und einem Antrieb (7) zur Erzeugung einer Relativverschiebung zwischen dem Röntgenaufnahmesystem und dem Patientenlagerungstisch oder Teilen desselben, gekennzeichnet durch

- Steuermittel zum Steuern des Aufnahmezeitpunktes (9) und/oder des Antriebs (7) derart, daß der Kontrastmittelbolus in den Röntgenaufnahmen abgebildet wird,
- einen ersten Speicher (17) zur Aufnahme von Zeilen ohne Kontrastmittel und einen zweiten Speicher zur Aufnahme von Zeilen mit Kontrastmittel und durch
- eine Einheit (10), die aus den Bildzeilen im ersten bzw. im zweiten Bildspeicher das Maskenbild bzw. das Kontrastmittelbild erstellt.

**3.** Anordnung nach Anspruch 2, dadurch gekennzeichnet, daß eine Wiedergabeeinheit (14) zur Beobachtung des Kontrastmittelflusses vorgesehen ist und daß Mittel (15) zum Steuern des Antriebs (7) und/oder des Aufnahmezeitpunkts durch den Benutzer vorgesehen sind.

**4.** Anordnung nach Anspruch 2, dadurch gekennzeichnet, daß die Einheit (10) Bildinterpretationsmittel zur Detektion der Kontrastmittelgrenze bzw. von Zeilen mit bzw. ohne Kontrastmittel enthält.

**5.** Anordnung nach Anspruch 2, dadurch gekennzeichnet, daß Mittel zum Bestimmen der Kontrastmittelgeschwindigkeit aus aufeinanderfolgenden Röntgenaufnahmen und zum Bestimmen des Aufnahmezeitpunktes und/oder der die Geschwindigkeit des Antriebs für die nachfolgende Röntgenaufnahme vorgesehen sind.

**6.** Anordnung nach Anspruch 2, dadurch gekennzeichnet, daß im Strahlengang des Röntgenaufnahmesystems Marken (201, 202) vorgesehen sind, die in einer Röntgenaufnahme abbildbar sind.

Fig.1

Fig.2

Fig.3a

Fig.3b